Europäisches Patentamt

European Patent Office

Office européen des brevets

(11) Veröffentlichungsnummer: **0 293 789**
A1

## (12) EUROPÄISCHE PATENTANMELDUNG

(21) Anmeldenummer: 88108551.8

(22) Anmeldetag: 28.05.88

(51) Int. Cl.⁴: **B66C 6/00** , **E04C 3/07**

(30) Priorität: 29.05.87 DE 3718101
15.07.87 DE 3723324

(43) Veröffentlichungstag der Anmeldung:
07.12.88 Patentblatt 88/49

(84) Benannte Vertragsstaaten:
AT BE CH DE ES FR GB IT LI NL SE

(71) Anmelder: Scheffer Maschinen- u.
Apparatebau GmbH
Füchtorfer Strasse 60
D-4414 Sassenberg(DE)

(72) Erfinder: Scheffer, Heinz
Füchtorfer Strasse 60
D-4414 Sassenberg(DE)

(74) Vertreter: Habbel, Hans-Georg, Dipl.-Ing.
Postfach 3429 Am Kanonengraben 11
D-4400 Münster(DE)

(54) Als Kastenträger ausgebildeter Kranträger.

(57) Die bisher bekannten kastenförmigen Kranträger sind im Schnitt rechteckig ausgebildet und weisen daher häufig nicht die erforderliche Verwindungssteifigkeit bei außermittiger Belastung auf.

Eine Verwindungssteifigkeit auch bei großer Länge des Kastenträgers wird dadurch erreicht, daß die beiden seitlichen Stegbleche als konkav gewölbte Schalen ausgebildet sind.

Fig. 1

EP 0 293 789 A1

"Als Kastenträger ausgebildeter Kranträger"

Die Erfindung bezieht sich auf einen Kranträger gemäß dem Oberbegriff des Hauptanspruches.

Derartige Kranträger sind aus der DE-OS 35 14 786 bekannt. Bei diesem gattungsbildenden Kranträger, der als Kastenträger gestaltet ist, schließen die beiden seitlichen Stegbleche an den Ober- und Untergurt unter einem Winkel von 90° an, d.h. es wird tatsächlich ein im Querschnitt rechteckiger Kasten geschaffen.

Wird ein solcher Kasten durch aufgesetzte Kranschienen oder untergehängte Laufkatzen außermittig belastet, ist ein Verwinden des Kastens nicht auszuschließen, trotz der vorgesehenen Innenversteifungen.

Die bekannten Kranträger weisen häufig eine Länge von 30 bis 40 m auf und bei der gattungsbildenden bekannten Anordnung ist ein Flattern oder Ausbeulen nur dadurch auszuschalten, daß zusätzliche Versteifungen eingebaut werden.

Der Erfindung liegt die Aufgabe zugrunde, einen Kranträger zu schaffen, der als Kastenträger ausgebildet ist, kostengünstig hergestellt werden kann und bei außermittiger Belastung trotzdem die erforderliche Steifigkeit und Verwindungssicherheit aufweist.

Diese der Erfindung zugrundeliegende Aufgabe wird durch die Lehre des Hauptanspruches gelöst.

Vorteilhafte Ausgestaltungen sind in den Unteransprüchen erläutert.

Mit anderen Worten ausgedrückt, schlägt die Erfindung einen kastenförmigen Kranträger vor, bei welchem die beiden seitlichen Stegbleche als nach außen konkav gewölbte oder profilierte Schalen ausgebildet sind, wobei sich diese rinnenartigen Schalen über die ganze Länge des Kastenträgers erstrecken. Überraschend wurde festgestellt, daß bei einer solchen Gestaltung der seitlichen Stegbleche eine bisher nicht geahnte Festigkeit in den Kranträger eingetragen wird, was bewirkt, daß der Kranträger trotz außermittiger Belastung sich nicht verwindet, wobei die gewölbte Gestaltung besondere Festigkeiten erreicht.

Wenn eine zusätzliche Versteifung notwendig ist, ist die einfachste Aussteifungsmöglichkeit für die beiden Stegbleche ein in dem Innenraum des durch die beiden Stegbleche gebildeten Kastens eingesetztes Versteifungsdreieck, das mit seinem Scheitel an dem Scheitel der einen Mulde des seitlichen Stegbleches anschließt und mit den Schenkeln auf der Innenseite des anderen Stegbleches aufsteht und hier befestigt ist.

In den Innenraum kann als Versteifung auch ein ka stenförmiges Profilbauteil eingesetzt sein, das mit der Innenseite der seitlichen Stegbleche verschweißt oder verschraubt werden kann. Die geschraubte Ausführung ist dabei wesentlich wirtschaftlicher als die geschweißte, da diese kastenförmigen Profilbauteile in Serie vorgefertigt werden können. Als zusätzlicher Vorteil tritt dabei ein, daß beim Zusammensetzen keine Verspannungen auftreten, die andernfalls durch die hohen Schweißtemperaturen erzeugt werden.

Auch ist es möglich, ohne Innenversteifung zu arbeiten, aber eine zusätzliche Festigkeit dadurch zu erreichen, daß sich die beiden Scheitel der Stegbleche berühren.

In weiterer Ausbildung der Erfindung ist es möglich, daß die Versteifung auch außen an die beiden Stegbleche angesetzt wird, so daß also außen auf die beiden Stegbleche Stützbleche aufgesetzt werden, die vorzugsweise mit den Stegblechen dann verschraubt sind.

Werden abgekantete Stegbleche eingesetzt, kann der durch die Kantung gebildete Winkel zwischen 165 bzw. 150° liegen, wobei bei einem Winkel von 165° sehr gute Festigkeitsergebnisse erzielt wurden.

Überraschend wurde zudem festgestellt, daß trotz erheblicher Längen derart ausgebildeter Kastenträger ein Flattern und Ausbeulen der seitlichen Stegbleche nicht mehr eintritt.

Ausführungsbeispiele der Erfindung werden nachfolgend anhand der Zeichnungen erläutert. Die Zeich nungen zeigen in

Fig. 1 eine erste Ausführungsform des Kranträgers mit gewölbten seitlichen Stegblechen, in

Fig. 2 die Gestaltung des Kranträgers gemäß Fig. 1 aber mit eingesetztem kastenförmigen Hohlprofilteil, in

Fig. 3 eine abgeänderte Ausführungsform des eingesetzten kastenförmigen Hohlprofilteils, in

Fig. 4 einen Kranträger mit seitlichen Stegblechen, die als Winkelprofilteile ausgebildet sind und in

Fig. 5 die Ausführungsform gemäß Fig. 4 aber mit abgeändertem kastenförmigen Hohlprofilteil als Versteifung.

In den Zeichnungen ist mit 1 ein Obergurt und mit 2 ein Untergurt bezeichnet. Obergurt 1 und Untergurt 2 werden durch zwei seitliche Stegbleche 3 und 4 miteinander verbunden.

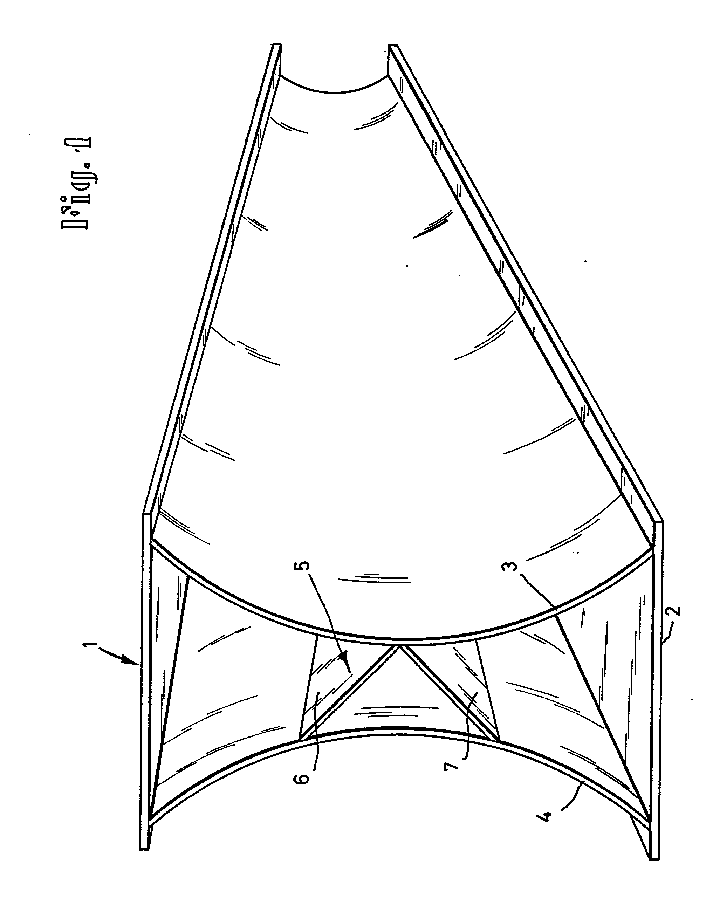

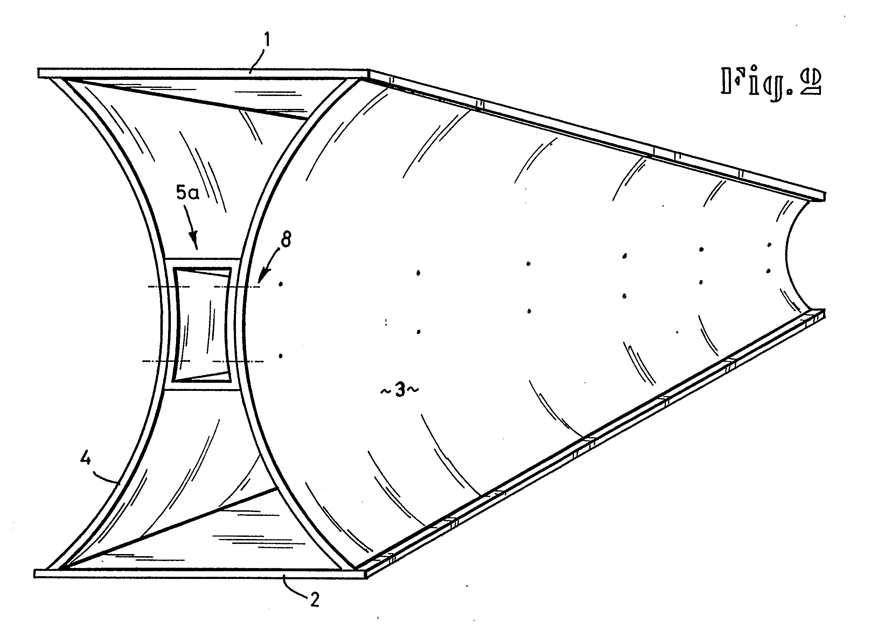

Die beiden seitlichen Stegbleche 3 und 4, die als Höckerbleche ausgebildet sein können, sind gemäß Fig. 1 und 2 als Schalenbleche ausgebildet und konkav zwischen Obergurt 1 und Untergurt 2 eingeschaltet und schließen damit mit ihren Ober- und Unterkanten etwa im Außenbereich von Obergurt 1 und Untergurt 2 an, während im mittleren Bereich der Abstand zwischen den beiden Schalenblechen geringer als im übrigen Bereich ist.

In diesen mittleren Bereich ist eine Versteifung 5 bzw. 5a eingeschaltet, die bei dem in Fig. 1 dargestellten Ausführungsbeispiel aus einem dreieckförmigen Gebilde besteht, das durch die beiden Bleche 6 und 7 geschaffen wird. Diese beiden Bleche 6 und 7 schließen unter einem Winkel von 90° aneinander an und der so gebildete Scheitel des Dreiecks ist an der Rückseite des seitlichen Stegbleches 3 befestigt. Die Schenkel der Versteifung 3 schließen an der Rückseite des seitlichen Stegbleches 4 an und sind hier ebenfalls verschweißt. Vorteilhaft ist es, wenn das untere Versteifungsblech 7 stärker gewählt wird als das obere.

In Fig. 2 ist die Versteifung 5a als kastenförmiges Hohlprofilteil ausgebildet, das - wie dies durch die Linien 8 verdeutlicht wird - mit den seitlichen Stegblechen 3 und 4 verschraubt ist.

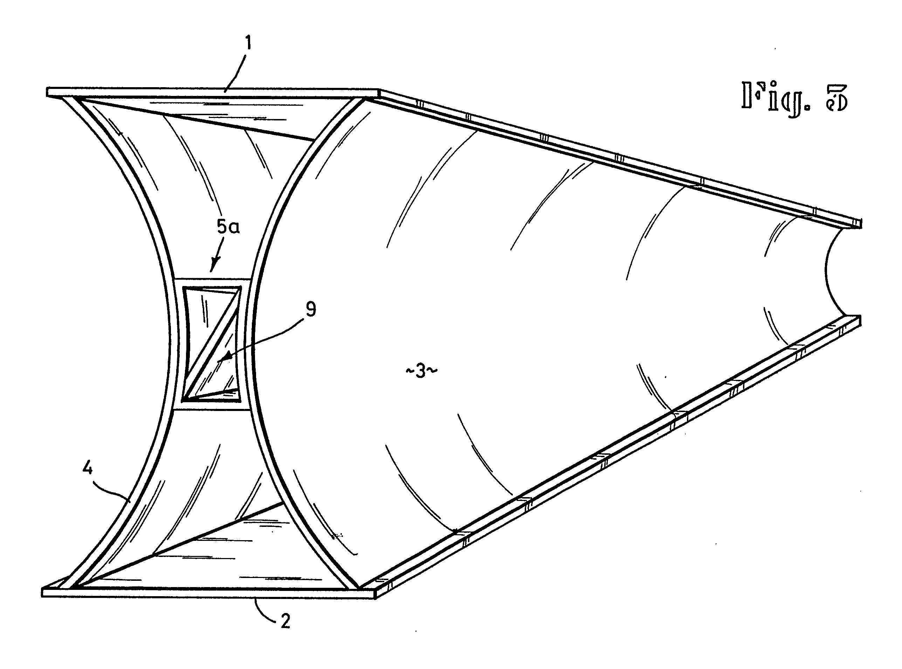

Bei der Ausführungsform gemäß Fig. 3 ist das kastenförmige Hohlprofilteil, welches die Versteifung 5a bildet, in den Innenraum der seitlichen Stegbleche 3 und 4 eingeschweißt und weist zusätzlich eine Querversteifung 9 auf.

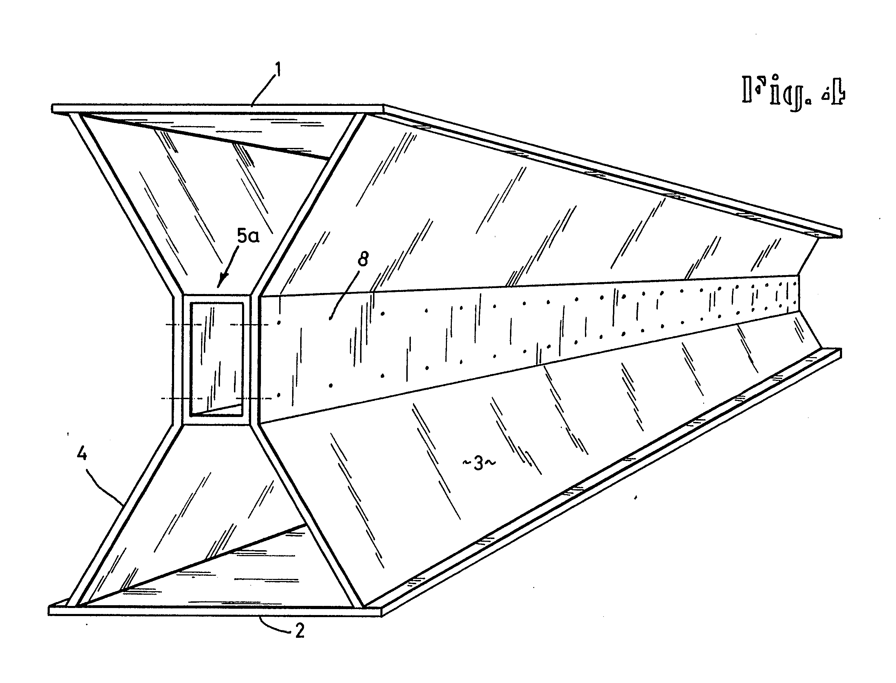

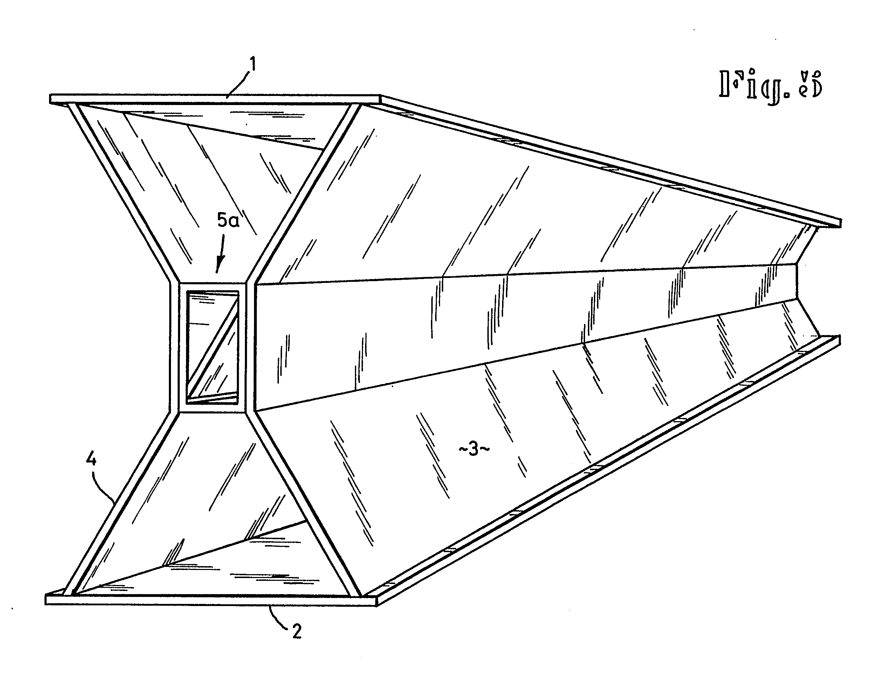

Bei den Ausführungsformen gemäß Fig. 4 und 5 sind die seitlichen Stegbleche 3, 4 als Winkelprofilteile ausgebildet. Bei diesen Ausführungsbeispielen ist ebenfalls in den Innenraum eine Versteifung 5a eingesetzt, die als kastenförmiges Hohlprofilteil ausgebildet ist.

Die verschraubte Ausführungsform gemäß den Fig. 2 und 4 hat gegenüber der eingesetzten und verschweißten Versteifung den Vorteil, daß sie wesentlich wirtschaftlicher ist. Die durch Verschrauben eingesetzten und gehaltenen Versteifungen 5a können in Serie vorgefertigt werden. Beim Zusammenbau des Kranträgers werden außerdem Verspannungen vermieden, die durch die Schweißtemperaturen andernfalls erzeugt werden.

Über den erzielten technischen und wirtschaftlichen Vorteil hinaus, ist der erfindungsgemäße Kranträger zudem formschön gestaltet und weist keine, über die Außenseite der seitlichen Stegbleche vorstehenden, zu Schmutzanlagerungen führenden Bereiche auf.

In den Zeichnungen nicht dargestellt ist die Möglichkeit, die Versteifung auch dadurch zu erreichen, daß außen auf die Stegbleche 3, 4 Versteifungsbleche aufgesetzt werden, die dem Kantprofil bzw. dem Wölbungsprofil der Stegbleche entsprechen, wobei dann die Stegbleche an der Innenseite aneinanderstoßen und die Versteifung außen angeordnet ist, die mit den Stegblechen verschweißt oder verschraubt sein kann.

Auch ist in den Zeichnungen bewußt die Ausführungsform nicht dargestellt, bei der auf jede Innenversteifung verzichtet wird und die Stegbleche an mindestens einer Kante oder einer der Flächen zwischen den Abkantungen oder Wölbungen Kontakt miteinander haben. Durch diese Maßnahme werden besondere Abstützelemente zwischen zwei Stegblechen, z. B. in Form von Blechen oder Ausschäumungen, vermieden.

Zusammenfassend ist festzustellen, daß durch diese Gestaltung der Stegbleche eine faltwerkartige Ausbildung des Stegbleches mit Einzelfeldern erzielt wird, die durch die Kanten oder Wölbungen ausgesteift sind.

## Ansprüche

1. Als Kastenträger ausgebildeter Kranträger mit einem Obergurt (1), einem Untergurt (2) und zwischen Ober- und Untergurt angeordneten seitlichen Stegblechen (3, 4), dadurch gekennzeichnet, daß die seitlichen Stegbleche (3, 4) von außen gesehen als konkav gestaltete langgestreckte Schalenbleche ausgebildet sind.

2. Kranträger nach Anspruch 1, dadurch gekennzeichnet, daß die konkav gestalteten Stegbleche (3, 4) konkav gewölbt ausgebildet sind (Fig. 1 bis 3).

3. Kranträger nach Anspruch 1, dadurch gekennzeichnet, daß die konkav gestalteten Stegbleche (3, 4) als Winkelprofilteile ausgebildet sind (Fig. 4, 5).

4. Kranträger nach einem der Ansprüche 1 bis 3, dadurch gekennzeichnet, daß die Stegbleche (3, 4) über eine in Längsrichtung des Kastenträgers verlaufende Versteifung miteinander verbunden sind.

5. Kranträger nach einem der Ansprüche 1 bis 4, dadurch gekennzeichnet, daß die Versteifung (5) im Inneren der beiden seitlichen konkav gewölbten Stegbleche (3, 4) aus zwei sich in Längsachse des Kranträgers erstreckenden Blechen (6, 7) ausgebildet ist, die im Schnitt gesehen ein Dreieck bilden, dessen Scheitel an der Rückseite des einen Stegbleches (3) angreift, während die Schenkel im Endbereich an der Rückseite des anderen Stegbleches (4) befestigt sind (Fig. 1).

6. Kranträger nach einem der Ansprüche 1 bis 5, dadurch gekennzeichnet, daß der Winkel zwischen den beiden Blechen (6, 7) etwa 90° beträgt.

7. Kranträger nach einem der Ansprüche 1 bis 6, dadurch gekennzeichnet, daß das untere Blech (7) der Versteifung (5) stärker als das obere Blech (6) der Versteifung (5) ausgebildet ist.

8. Kranträger nach einem oder mehreren der Ansprüche 1 bis 7, dadurch gekennzeichnet, daß die Versteifung (5a) als kastenförmiges Hohlprofilteil ausgebildet ist (Fig. 2 bis 4).

9. Kranträger nach Anspruch 8, dadurch gekennzeichnet, daß die als kastenförmiges Hohlprofilteil ausgebildete Versteifung (5a) in den Innenraum der beiden seitlichen Stegbleche (3, 4) im Bereich des Scheitels derselben angeschraubt (bei 8) ist (Fig. 2 und 4).

10. Kranträger nach Anspruch 8, dadurch gekennzeichnet, daß die durch das kastenförmige Hohlprofilteil gebildete Versteifung (5a) in den Innenraum der beiden seitlichen Stegbleche (3, 4) eingeschweißt ist (Fig. 3 und 5).

11. Kranträger nach einem oder mehreren der vorhergehenden Ansprüche, dadurch gekennzeichnet, daß in die durch ein kastenförmiges Hohlprofilteil gebildete Versteifung eine Querversteifung (9) eingesetzt ist (Fig. 3 und 5).

12. Kranträger nach einem der Ansprüche 1 bis 3, dadurch gekennzeichnet, daß sich die Scheitel der Stegbleche (3, 4) berühren und miteinander verbunden sind.

13. Kranträger nach einem oder mehreren der vorhergehenden Ansprüche, dadurch gekennzeichnet, daß auf die Stegbleche (3, 4) außen Versteifungen im Bereich der Wölbung oder Faltkante aufgesetzt sind, die durch Verschweißen oder Verschrauben festgelegt sind.

Fig. 1

Fig. 2

Fig. 5

EP 0 293 789 A1

Fig. 4

Fig. 1

EP 0 293 789 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-3 241 285 (BARONI) <br> * Spalte 1, Zeilen 11-20; Spalte 8, Zeilen 42-74; Figuren 1-9 * | 1,2,4 | B 66 C 6/00 <br> E 04 C 3/07 |
| Y | | 8,10 | |
| | --- | | |
| X | FR-A-1 256 094 (LACOUR) <br> * Insgesamt * | 1,2 | |
| | --- | | |
| X | FR-A-1 467 255 (LE METAL DEPLOYE) <br> * Seite 1, rechte Spalte, letzter Absatz * | 1,2,12 | |
| | --- | | |
| X | US-A-1 989 834 (WATSON) <br> * Insgesamt * | 1,3,4 | |
| | --- | | |
| X | FR-A-2 110 360 (BOONEN) <br> * Insgesamt * | 1,3,12 | |
| | --- | | |
| X | DE-A-2 205 093 (LIEBHERR-WERK EHINGEN) <br> * Seite 6, letzter Absatz; Figur 8 * | 1,3,13 | |
| | --- | | RECHERCHIERTE SACHGEBIETE (Int. Cl.4) |
| Y | FR-A-1 384 434 (MALCUS HOLMQUIST) <br> * Insgesamt * | 8,10 | B 66 C <br> E 04 C |
| | --- | | |
| A | DE-A-2 317 595 (KLAUS) | | |
| | --- | | |
| A | GB-A-1 075 774 (ISHIKAWAJIMA-HARIMA JUKOGYO K.K.) | | |
| | --- | | |
| A | CH-A- 436 646 (DILL) | | |

Der vorliegende Recherchenbericht wurde für alle Patentansprüche erstellt

| Recherchenort | Abschlußdatum der Recherche | Prüfer |
|---|---|---|
| DEN HAAG | 08-09-1988 | VAN DEN BERGHE E.J.J. |

EPO FORM 1503 03.82 (P0403)